# DEMANDE DE BREVET EUROPEEN

(11) **EP 1 085 082 A1**
(43) Date de publication de la demande: **21.03.2001**
(21) Numéro de dépôt: 00402537.5
(22) Date de dépôt: 14.09.2000
(51) Int. Cl.: C12N 5/02, C12N 5/06, C12N 5/08, A61K 35/32, A61P 19/00, A61K 35/12, A61L 27/38

(54) **Méthodes et compositions pour la conservation de chondrocytes et utilisations**

(30) Priorité: 16.09.1999 FR 9911564
(71) Demandeur: UNIVERSITE PIERRE ET MARIE CURIE PARIS VI, 75252 Paris Cédex 05 (FR)
(72) Inventeur: Salzmann, Jean-Loup, 75005 Paris (FR); Crespo, Andrès, 94490 Ormesson sur Marne (FR); Klatzmann, David, 75013 Paris (FR)
(74) Mandataire: Becker, Philippe

(57) **Abrégé**

La présente invention concerne des compositions de cellules chondrocytaires, notamment humaines, et des méthodes pour leur préparation et leurs utilisations. L'invention décrit plus particulièrement la production de suspensions de chondrocytes humains autologues, utilisant des enzymes recombinantes et/ou des milieux compatibles avec un usage pharmaceutique. L'invention décrit également des méthodes et compositions pour la congélation de chondrocytes, notamment en l'absence de DMSO. Les chondrocytes produits peuvent être utilisés in vivo pour restaurer des structures cartilagineuses affectées, telles que des défauts cartilagineux post-traumatiques ou l'ostéochondrite disséquante du genou ou, plus généralement, pour le traitement et la réfection des défauts cliniquement significatifs du cartilage, notamment du cartilage articulaire.

## Description

La présente invention concerne des compositions de cellules chondrocytaires, notamment humaines, et des méthodes pour leur préparation. Elle concerne également l'utilisation de ces compositions pour l'implantation de chondrocytes in vivo, pour le traitement de différentes pathologies. L'invention décrit plus particulièrement la production de suspensions de chondrocytes humains autologues, des techniques pour leur conservation, et leur utilisation in vivo pour restaurer des structures cartilagineuses affectées, telles que des défauts cartilagineux post-traumatiques ou l'ostéochondrite disséquante du genou ou, plus généralement, pour le traitement et la réfection des défauts cliniquement significatifs du cartilage, notamment du cartilage articulaire.

Les lésions chondrales, telles que par exemple les lésions focales du cartilage articulaire du genou en zone portante, exposent fortement les sujets à l'arthrose. Ce type de lésions est fréquent (traumatisme, activité sportive, ostéochondrite disséquante, etc.) et occasionne une gêne importante. Les capacités de réparation spontanée des lésions chondrales sont faibles. Celles-ci peuvent être caractérisées par la présence de cicatrices fibro-cartilagineuses, par étude arthroscopique. Ces lésions sont essentiellement traitées par lavage, ce qui guérit les symptômes de manière temporaire, mais ne traite pas l'origine du défaut et, en particulier, n'évite pas une possible dégradation progressive du cartilage. Cette évolution arthrosique du cartilage ne semble en réalité pouvoir être traitée efficacement que par remplacement du cartilage défectueux par un cartilage sain. En particulier, l'utilisation d'autres cellules que les chondrocytes entraîne une réparation inefficace du cartilage, en changeant la nature de la matrice extracellulaire.

A cet effet, la production et l'utilisation thérapeutique de chondrocytes humains, en particulier autologues, pour reconstituer ou compenser des défauts du cartilage ont été décrites dans la littérature.

Ainsi, il est connu d'isoler des chondrocytes à partir de différents types de cartilage ou même de précurseurs issus de la moelle osseuse. Pour cela, un échantillon de cartilage sain (ou de moelle) peut être prélevé chez un sujet, disséqué mécaniquement pour former des fragments de taille réduite, puis traité en présence d'une ou plusieurs enzymes, généralement la trypsine et/ou une collagénase d'extraction, afin de séparer les chondrocytes inclus dans le cartilage. Les chondrocytes peuvent ensuite être cultivés, généralement en monocouche, de manière à obtenir un nombre suffisant de cellules (plusieurs passages sont généralement réalisés). Bien que les chondrocytes se dédifférencient sous forme de fibroblastes lors de ces étapes de culture et d'expansion, il a été montré que les cellules chondrogéniques obtenues pouvaient se différencier à nouveau en cellules chondrocytes fonctionnelles, soit in situ, soit in vitro par ensemencement dans des cultures tridimensionnelles.

Différentes approches ont été développées pour la culture in vitro des chondrocytes, et différentes stratégies de traitement ont été élaborées. En particulier, la demande internationale n° WO95/30742 propose de reconstituer in vitro des patchs de cartilage artificiel (c'est-à-dire des chondrocytes multicouches inclus dans une matrice extracellulaire endogène) de forme et volume prédéfinis, et d'implanter ces patchs sur les sites à traiter. D'autres stratégies reposent sur la production de cultures de chondrocytes (ou de cellules chondrogéniques) expansées in vitro, qui sont implantées directement afin de reconstituer un cartilage in situ, ou après dépôt sur des matrices synthétiques biocompatibles. L'utilisation de facteurs de stimulation de la croissance des précurseurs des cellules chondrocytes a également été suggérée, afin d'induire la production de chondrocytes in situ.

La présente invention décrit à présent de nouvelles compositions et méthodes permettant d'améliorer les conditions de production et d'utilisation de chondrocytes, pour des applications thérapeutiques. La présente invention décrit en particulier de nouvelles méthodes permettant une production améliorée de chondrocytes. Notamment, la présente invention décrit des techniques de production de chondrocytes permettant de générer des préparations de chondrocytes de qualité pharmaceutique. La présente invention décrit également de nouvelles techniques permettant de conserver efficacement les chondrocytes, notamment sous forme congelée, ce qui facilite considérablement les conditions de mise en oeuvre d'un programme clinique de greffe. La présente invention peut être mise en oeuvre pour générer des suspensions de chondrocytes autologues humains, destinés à être implantés in vivo, ou pour produire des patchs de cartilage artificiels, ou également pour la préparation de matrices synthétiques recouvertes de chondrocytes. La présente invention décrit également des méthodes pour l'implantation des chondrocytes dans les zones à traiter (notamment les cartilages).

Un premier aspect de l'invention réside donc dans la production de chondrocytes à partir d'un échantillon biologique, en particulier de chondrocytes en culture à partir d'un échantillon biologique comprenant des chondrocytes inclus dans une matrice extracellulaire cartilagineuse, encore plus préférentiellement de chondrocytes en suspension à partir d'une biopsie de tissu cartilagineux. Les procédés de l'invention comprennent différentes étapes de traitement et conditionnement de l'échantillon biologique, puis de culture et de traitement des chondrocytes, permettant d'obtenir des préparations de chondrocytes de qualité pharmaceutique, c'est-à-dire utilisable en thérapeutique humaine.

Pour une meilleure compréhension de la présente invention, les définitions suivantes sont fournies :
Cartilage (ou tissu cartilagineux) : Le cartilage est essentiellement formé de chondrocytes, incorporés dans une matrice extracellulaire. La matrice extracellulaire comprend principalement du collagène (essentiellement de type II) et des protéoglycans (essentiellement des protéines sur lesquelles sont liées des glycosaminoglycans tels les chondroitines sulfate et les kératan sulfate). La matrice extracellulaire est sérétée par les chondrocytes. Il existe différents types de cartilages, tels que le cartilage hyalin (présent notamment dans les articulations, en particuler le cartilage costal, nasal, bronchial, etc.), le cartilage élastique, qui renferme en plus des fibres d'élastine (présent par exemple dans l'oreille) et le cartilage fibreux, qui renferme en plus du collagène de type I (présent notamment dans les disques intervertébraux, le ménisque, etc.). Au sens de l'invention, le terme cartilage désigne tous les types de cartilages, comprenant notamment des chondrocytes et une matrice extracellulaire.

Les chondrocytes sont les cellules du cartilage qui sécrètent la matrice extracellulaire. Au sens de la présente invention, le terme chondrocyte désigne plus spécifiquement les cellules qui sécrètent la matrice extracellulaire cartilagineuse, et notamment le collagène de type II et des protéoglycans tels les agrécannes. Le terme chondrocytes inclut également, au sens de la présente invention, les cellules « chondrogéniques », c'est-à-dire les cellules susceptibles de produire des chondrocytes. Il s'agit donc de cellules susceptibles de produire la matrice extracellulaire cartilagineuse, telles que des précurseurs des chondrocytes, notamment de précurseurs mésenchymateux (ou les cellules souches mésenchymateuses), ou des chondrocytes dédifférenciés, par exemple à la suite de cultures en monocouches. Le terme chondrocyte désigne aussi bien des cultures primaires (fraichement isolées de biopsies) que des cellules expansées in vitro, voire modifiées génétiquement, immortalisées, sélectionnées, conservées, etc.

Selon une première caractéristique avantageuse, la méthode de production selon l'invention comprend une étape a) de traitement de l'échantillon biologique en présence d'une enzyme recombinante permettant la dissociation des chondrocytes, en particulier par hydrolyse (au moins partielle) de molécules de collagène contenues dans l'échantillon.

Comme il sera expliqué en détails plus loin, une première caractéristique du procédé de l'invention réside dans l'emploi d'une enzyme recombinante pour la dissociation de l'échantillon biologique, notamment de la biopsie de cartilage. La présente invention montre en effet que l'utilisation d'une telle enzyme améliore non seulement la qualité du produit, mais également l'efficacité du procédé.

L'invention a donc également pour objet un procédé de dissociation des chondrocytes contenus dans un échantillon biologique, comprenant le traitement in vitro de cet échantillon biologique en présence d'une enzyme recombinante capable d'hydrolyser les molécules de collagène, en particulier d'une collagénase recombinante.

L'invention a également pour objet l'utilisation d'une enzyme recombinante capable d'hydrolyser les molécules de collagène, en particulier d'une collagénase recombinante, pour dissocier les chondrocytes humains in vitro.

Dans le procédé de l'invention, les chondrocytes ainsi dissociés de l'échantillon biologique sont, dans une étape b), cultivés en monocouche afin de les multiplier (ou expandre), ou ensemencés directement au sein d'un gel dans le but de les multiplier. Lors de cultures en monocouche, les chondrocytes adhèrent à la surface du matériel de culture utilisé. En fin de culture, les cellules sont donc détachées pour être récoltées, puis, le cas échéant, mises en suspension. Dans le procédé de l'invention, les cellules peuvent être détachées par toute technique connue de l'homme du métier (en particulier, un traitement à la trypsine). Toutefois, selon une autre caractéristique avantageuse du procédé de l'invention, les chondrocytes sont détachés par traitement des cellules en présence d'une solution comprenant au moins un composé choisi parmi les dérivés polyosidiques. En effet, un deuxième aspect de la présente invention réside également dans la mise en évidence que les chondrocytes peuvent être détachés efficacement de leur support, sans affecter leurs propriétés fonctionnelles, par mise en contact avec un milieu comprenant des composés particuliers, choisis parmi les dérivés polyosidiques. L'utilisation de ce type de composés peut s'avérer particulièrement avantageuse pour des applications thérapeutiques, dans le mesure où ces composés ne présentent pas de pathogénicité reconnue ou sont déjà, pour certains d'entre eux, utilisés comme produits pharmaceutiques.

Un autre aspect de l'invention réside donc dans un procédé pour détacher des chondrocytes adhérents en culture in vitro, comprenant la mise en contact des chondrocytes avec un milieu comprenant au moins un composé choisi parmi les dérivés polyosidiques (notamment l'héparine).

L'invention a également pour objet l'utilisation d'un composé choisi parmi les dérivés polyosidiques, en particulier de l'héparine, pour détacher les chondrocytes en culture

Dans un mode de mise en oeuvre plus spécifique, le procédé de production d'une préparation de chondrocytes selon l'invention comprend donc :
a) le traitement de l'échantillon biologique en présence d'une enzyme recombinante permettant la dissociation des chondrocytes,
b) la culture en monocouche des chondrocytes ainsi dissociés, et
c) la mise en contact des chondrocytes avec un milieu comprenant au moins un composé choisi parmi les dérivés polyosidiques, afin de détacher les chondrocytes adhérents.

Dans une autre variante de l'invention, le procédé de production d'une préparation de chondrocytes comprend:
a) le traitement de l'échantillon biologique en présence d'une enzyme permettant la dissociation des chondrocytes,
b) la culture en monocouche des chondrocytes ainsi dissociés, et
c) la mise en contact des chondrocytes avec un milieu comprenant au moins un composé choisi parmi les dérivés polyosidiques, afin de détacher les chondrocytes adhérents.

Dans le procédé de production de l'invention, les étapes b) et c) décrites ci avant peuvent être répétées plusieurs fois, de manière à générer un nombre important de cellules chondrocytes à partir de l'échantillon biologique, chaque cycle répété étant désigné communément par le terme « passage ». La répétition des étapes b) et c), et l'expansion des chondrocytes qui en résulte, sont particulièrement importantes pour des application de greffes autologues, où il est essentiel de produire une quantité suffisante de chondrocytes, à partir d'une biopsie, pour pallier au défaut cartilagineux du patient.

Lorsque les cellules ont été détachées du support, elles sont ensuite récupérées dans tout dispositif approprié (fiole, tube, flacon, poche, etc.). Généralement, la densité cellulaire est déterminée, pour évaluer la quantité de cellules disponibles. La viabilité cellulaire et la stérilité sont également testées pour vérifier la qualité de la préparation obtenue.

Dans un mode de mise en oeuvre particulier, le procédé de production d'une préparation de chondrocytes selon l'invention comprend donc :
a) le traitement de l'échantillon biologique en présence d'une enzyme recombinante permettant la dissociation des chondrocytes,
b) la culture en monocouche des chondrocytes ainsi dissociés,
c) la mise en contact des chondrocytes avec un milieu comprenant au moins un composé choisi parmi les dérivés polyosidiques, afin de détacher les chondrocytes adhérents,
d) le cas échéant, la répétition une ou plusieurs fois des étapes b) et c), et,
e) la récupération des cellules produites.

Dans une autre de l'invention, le procédé de production d'une préparation de chondrocytes comprend:
a) le traitement de l'échantillon biologique en présence d'une enzyme permettant la dissociation des chondrocytes,
b) la culture en monocouche des chondrocytes ainsi dissociés,
c) la mise en contact des chondrocytes avec un milieu comprenant au moins un composé choisi parmi les dérivés polyosidiques, afin de détacher les chondrocytes adhérents,
d) le cas échéant, la répétition une ou plusieurs fois des étapes b) et c), et,
e) la récupération des cellules produites.

Les cellules ainsi produites peuvent être mises en suspension dans tout milieu désiré et utilisées telles quelles, comme produit thérapeutique. En effet, l'implantation de suspensions de chondrocytes, notamment autologues, pour des applications cliniques dans la régénération du tissu cartilagineux à été rapportée dans la littérature. Ces cellules peuvent également être utilisées pour des applications de recherche et d'étude sur le développement et la biologie des chondrocytes, pour la production de banques d'acides nucléiques de chondrocytes, etc. Ces cellules peuvent également être manipulées, par exemple pour y introduire des acides nucléiques hétérologues. Les cellules peuvent enfin être ensemencées dans des cultures tridimensionnelles ou dans des supports non-adhérents, pour former in vitro des matrices à implanter ou des cartilages artificiels.

A cet égard, pour faciliter l'ensemble de ces manipulation, et pour tout autre usage ultérieur, ces cellules peuvent également être conditionnées, notamment par congélation. A cet effet, la présente invention décrit également des compositions et méthodes particulièrement efficaces pour conserver les chondrocytes, notamment sous forme congelée. Ces méthodes permettent avantageusement de conserver les chondrocytes sur de longues périodes, sans affecter leurs propriétés fonctionnelles. En outre, les compositions et méthodes utilisées peuvent être compatibles avec un usage thérapeutique, et permettent donc de préserver les chondrocytes ou les compositions de chondrocytes destinées à être implantées in vivo.

Ainsi, dans un mode particulier de réalisation, le procédé de l'invention comprend en outre une étape f) de congélation des chondrocytes obtenus.

La congélation peut être réalisée dans différentes conditions, et en présence de différents agents ou compositions protecteurs. Ainsi, selon une première variante de réalisation, la congélation est réalisée en présence de diméthyl sulfoxyde (« DMSO »). Le DMSO est un agent protecteur bien connu, qui s'insère dans les membranes cellulaires et permet de les stabiliser, ce qui empêche la destruction des cellules. Ce type de milieu de congélation peut être mis en oeuvre avec les chondrocytes selon l'invention.

De manière particulièrement avantageuse, la congélation est réalisée en absence de DMSO. Plus particulièrement, la présente invention décrit à présent des méthodes et compositions pour la congélation des chondrocytes ou autres cellules eucaryotes, en conditions compatibles avec un usage thérapeutique direct (i.e., sans traitement des compositions décongelées).

L'invention concerne notamment tout milieu permettant la congélation de chondrocytes, dépourvu de DMSO et/ou de glycérol, et compatible avec un usage thérapeutique (c'est-à-dire administrable à un sujet sans étape préalable de lavage ou de centrifugation, par exemple). Plus particulièrement, il s'agit d'une composition comprenant au moins :
- de l'albumine humaine,
- une gélatine modifiée ou un polysaccharide, et
- éventuellement une solution saline.

A cet égard, l'invention décrit plus particulièrement la mise au point de nouveaux milieux pour la conservation de cellules eucaryotes, comprenant :
- de l'albumine humaine,
- un polysaccharide, et
- éventuellement une solution saline.

La présente invention montre en effet que de tels milieux peuvent être utilisés pour conserver des cellules eucaryotes, et pour les congeler sans affecter de manière substantielle leur viabilité. Ainsi, l'invention montre que, en adaptant les proportions respectives des constituants et la quantité de cellules, ces milieux peuvent être utilisés pour congeler des cellules eucaryotes, sans diminution substantielle de la viabilité des cellules à la décongélation, en particulier avec une diminution inférieure à 35% de la viabilité, plus préférentiellement inférieure à 25%, encore plus préférentiellement inférieure à 15%.

Dans ces milieux de conservation, le polysaccharide utilisé peut être de structure et de poids moléculaire variable. Préférentiellement, il s'agit d'un polysaccharide sulfaté, ayant de préférence un poids moléculaire compris entre 5000 et 500 000 dalton, plus préférentiellement entre 30 000 et 250 000 daltons. Le polysaccharide peut être choisi par exemple parmi le dextran (40 000 ou 60 000 daltons), l'amidon, l'hydroxyéthylamidon (240 000 dalton), etc.

La solution saline peut être toute solution isotonique telle que décrite dans la suite du texte.

L'albumine humaine peut être une albumine d'extraction ou une albumine recombinante, obtenue selon les techniques connues de l'homme du métier, comme décrit en détails dans la suite du texte.

A titre spécifique, on peut citer comme milieu selon l'invention des milieux comprenant de l'albumine humaine et une composition de substitut de plasma telle que l'Hémodex^{R}, l'Hestéril, le Plasmaclair^{R} ou encore le Rhéomacropdex^{R}. Ces produits sont utilisés en clinique et sont répertoriés dans le Vidal.

Dans ces milieux selon l'invention, les quantités respectives des constituants peuvent varier, selon le type cellulaire utilisé, la nature du polysaccharide, etc. Généralement, les milieux de l'invention comprennent de 5 à 45% d'une solution d'albumine humaine à 20% et de 95 à 55% de polysaccharide, éventuellement dans une solution saline.

Ces milieux selon l'invention, à base de polysaccharide et d'albumine, et dépourvus de DMSO (et préférentiellement de glycérol), peuvent être utilisés pour conserver et/ou congeler différents types de cellules eucaryotes, comme des cellules mammifère, notamment des cellules humaines. Il peut s'agir de cultures primaires, de lignées, de cellules modifiées génétiquement, de cellules productrices de virus, etc. A titre illustratif, on peut citer des cellules sanguines (érythrocytes, granulocytes, lymphocytes, cellules dendritiques, macrophages, etc.) des cellules souches embryonnaires ou somatiques, des cellules endothéliales, musculaires, hépatiques, nerveuses, fibroblastiques, chondrocytes, des cellules de production de rétrovirus, des cellules modifiées pour produire des composés thérapeutiques, etc. Dans un mode particulier, le milieu selon l'invention est mis en oeuvre pour la conservation de cellules présentatrices d'antigènes, notamment des monocytes, macrophages, cellules dendritiques ou leurs dérivés. Le milieu de l'invention peut en outre être mis en oeuvre pour conserver des concentrés de sang, ou d'érythrocytes, qui sont actuellement stockés en présence de DMSO ou de glycérol.

La présente invention décrit en outre des méthodes efficaces pour adapter la composition de tels milieux, voire améliorer leurs efficacité, comme il sera décrit en détails plus loin.

Dans un mode de réalisation particulier de l'invention, la congélation des chondrocytes est réalisée dans un milieu comprenant une solution saline, de l'albumine humaine et un dérivé de gélatine (en particulier une gélatine modifiée) ou un polysaccharide..

Un objet plus particulier de l'invention réside également dans une méthode de congélation de chondrocytes, notamment de chondrocytes humains, comprenant la mise en contact d'une préparation de chondrocytes, notamment de chondrocytes humains, avec un milieu comprenant une solution saline, de l'albumine humaine et un dérivé de gélatine ou un polysaccharide, de préférence un polysaccharide, et la congélation de la composition obtenue.

Plus préférentiellement, dans la méthode de congélation de l'invention, la préparation de chondrocytes, notamment de chondrocytes humains, comprend au moins environ 5.10⁶ cellules/ml, avantageusement au moins environ 10⁷ cellules/ml.

Un autre objet particulier de l'invention réside également dans une composition comprenant des chondrocytes, notamment des chondrocytes humains, une solution saline, de l'albumine humaine et un dérivé de gélatine ou un polysaccharide, de préférence un polysaccharide. Préférentiellement, de telles compositions selon l'invention comprennent au moins environ 5.10⁶ chondrocytes/ml, avantageusement au moins environ 10⁷ chondrocytes/ml.

L'invention concerne également toute composition comprenant des chondrocytes humains dédifférentiés in vitro et un véhicule pharmaceutiquement acceptable.

Les méthodes et préparations de chondrocytes selon l'invention, le cas échéant après décongélation, peuvent être utilisées in vitro ou in vivo, par implantation ou injection. Dans la suite du texte sera donnée une description plus détaillée des étapes du procédé de l'invention.

### Obtention de l'échantillon biologique

L'échantillon biologique utilisé pour la mise en oeuvre de l'invention peut être tout échantillon de tissu comprenant des chondrocytes. Il peut s'agir d'un fragment de tissu cartilagineux (hyalin, élastique ou fibreux), de moelle, ou de toute autre matière biologique comprenant des chondrocytes (y compris des précurseurs de chondrocytes).

Dans un mode préféré de mise en oeuvre de l'invention, l'échantillon biologique est un fragment de cartilage, de préférence un fragment de cartilage humain, encore plus préférentiellement un fragment de cartilage humain sain, en particulier autologue.

Le cartilage peut être prélevé dans différentes zones, telles que notamment au niveau d'articulations. A cet égard, dans un mode préféré de réalisation, l'échantillon biologique est un fragment de cartilage articulaire. Ce type de cartilage peut être prélevé en différentes zones telles que le condyle fémoral, l'échancrure, etc. Le prélèvement peut être réalisé plus spécifiquement au niveau de la partie supéro-externe du condyle fémoral ou bien de la zone létéro-externe de l'échancrure. De tels prélèvements permettent aisément d'obtenir des quantités suffisantes de chondrocytes primaires pour produire, selon le procédé de l'invention, des préparations de chondrocytes humains à la concentration de 20 à 30X10⁶ cellules/ml.

La biopsie est généralement prélevée dans une zone saine du cartilage, sous arthroscopie, de manière à obtenir une quantité supérieure ou égale à environ 100 mg de cartilage, de préférence au moins 150 mg de cartilage. Un prélèvement typique comprend environ 200 mg de cartilage au moins. Il est entendu que le cartilage utilisé pour le prélèvement de l'échantillon biologique est un tissu sain, comprenant des chondrocytes sains. A cet effet, les sujets donneurs sur lesquels sont effectués les prélèvements peuvent être soumis à différents tests sérologiques, notamment viraux (HIV, HBV, HCV, Hépatite, etc.). Généralement, dans le cadre d'une utilisation thérapeutique (greffe de chondrocytes), les critère d'exclusion des donneurs suivants sont utilisés : Femmes enceintes, séropositivité au VIH ou à l'hépatite, allergies à la gentamycine, antécédents de méniscectomie totale ou de rupture d'un ou des ligaments croisés, lésions en miroir sur le plan tibial, pathologie rotulienne (instabilité, chondropathie), désaxation frontale du genou supérieure à 10°, ou arthrose.

Pour le prélèvement, on procède par exemple à un carottage du cartilage du genou, sous arthroscopie, dans la zone supéro-externe du condyle fémoral, en prélevant toute l'épaisseur du cartilage. Après prélèvement, l'échantillon biologique peut être traité de différentes façons. Dans un premier mode de réalisation, l'échantillon biologique est utilisé directement dans le procédé de production de chondrocytes de l'invention. Il peut, dans ce but, être tout d'abord placé dans un milieu de transport, dans la situation où le prélèvement et le traitement de l'échantillon biologique sont réalisés en des endroits différents (par exemple l'hôpital pour le prélèvement et un laboratoire pour le traitement et la production des chondrocytes).

Dans un mode de réalisation particulier de l'invention, l'échantillon biologique est placé dans un milieu nutritif, à basse température (environ 4°C), pour conservation en vue d'une utilisation ultérieure. Dans ces conditions, il est possible de conserver l'échantillon biologique pendant plusieurs semaines. Ce mode de réalisation est particulièrement avantageux puisqu'il élimine les contraintes de temps rencontrées généralement dans les procédés de greffe de chondrocytes autologues. A cet égard, l'invention décrit et revendique à présent la possibilité de maintenir en culture l'échantillon biologique pendant un temps donné, préalablement à la phase de production des chondrocytes, sans altérer significativement les propriétés de l'échantillon, notamment la viabilité des chondrocytes qu'il contient.

Ainsi, dans un mode particulier, l'invention concerne également un procédé de production d'une culture de chondrocytes à partir d'un échantillon biologique, comprenant le prélèvement de l'échantillon biologique et son traitement pour produire les chondrocytes, caractérisé en ce que l'échantillon biologique est conservé en milieu nutritif préalablement à la phase de production des chondrocytes.

Plus particulièrement, l'échantillon biologique est placé, pendant une période pouvant aller de 1 à 15 semaines, voire plus, dans un milieu nutritif, de préférence à basse température (par exemple entre 2 et 8 °C environ, plus préférentiellement de l'ordre de 4°C environ ).

Dans un mode particulier, le procédé de l'invention tel que décrit ci- avant est donc caractérisé en ce que l'échantillon biologique est maintenu en culture dans un milieu nutritif, préalablement à l'étape a).

Le milieu nutritif utilisé pour conserver l'échantillon biologique peut être un milieu de culture adapté aux cellules ou tissus mammifère (DMEM, Eagle, RPMI, etc.). Le milieu peut être supplémenté de différents agents tels que des antibiotiques, acides aminés, minéraux, vitamine C, etc., et de sérum autologue ou de sérum humain AB sécurisé (c'est-à-dire prélevé à l'avance, testé pour la présence de contaminants viraux, le sujet donneur étant testé à nouveau pour la présence de tels contaminants plusieurs mois après. En outre, le milieu peut comprendre une enzyme capable de dissocier les cellules et/ou la matrice extracellulaire (en particulier d'hydrolyser le collagène), de manière à initier ou réaliser l'étape suivante a) du procédé de manière concomitante à celle-ci. Plus préférentiellement, il s'agit d'une collagénase recombinante, comme décrit ci-après. A cet égard, il peut s'agir d'une collagénase recombinante active à basse température, par exemple à 4°C. Une telle collagénase peut être préparée par mutagénèse dirigée selon les techniques connues de l'homme du métier. La composition finale et précise du milieu peut être adaptée par l'homme de l'art. Préférentiellement, l'échantillon biologique est placé dans une poche et conservé à 4°C, dans l'obscurité, éventuellement sous agitation lente.

### Etape a) : Traitement de l'échantillon biologique

L'échantillon biologique est traité selon le procédé de l'invention pour dissocier les chondrocytes qu'il renferme. Comme indiqué précédemment, la première étape du procédé comprend un traitement enzymatique de l'échantillon biologique, afin de désagréger l'échantillon et de récupérer les chondrocytes.

Préalablement au traitement enzymatique, l'échantillon biologique peut toutefois être conditionné, par exemple par traitement mécanique (découpé ou disséqué mécaniquement), afin d'obtenir une préparation tissulaire composée de fragments de taille réduite, notamment de l'ordre de 1 à 10 mm³, de préférence inférieure à 5 mm³. L'homogénéité de cette préparation n'est pas importante. Il est toutefois préférable que la majeure partie des fragments obtenus aient une dimension inférieure à 10 mm³, afin d'augmenter l'efficacité du procédé. Cette dissection de l'échantillon peut être réalisée par des méthodes connues, comme par exemple à l'aide d'une pince, scalpel, ciseaux, bistouri, tamisage sur des grilles, etc., en conditions stériles.

Le procédé de l'invention comprend donc une étape préalable de prélèvement de l'échantillon biologique suivie, le cas échéant, d'une étape de dissection mécanique de celui-ci.

L'échantillon biologique est ensuite soumis à un traitement en présence d'une composition comprenant au moins une enzyme recombinante capable d'hydrolyser le collagène, de préférence le collagène de type II ou, plus généralement, capable de dégrader un ou plusieurs constituants de la matrice extracellulaire cartilagineuse (composée principalement de collagène de type II, associé à des protéoglycans).

Une enzyme préférée pour cette étape est la collagénase. A cet égard, il est connu d'utiliser différentes collagénases bactériennes d'extraction (c'est-à-dire de forme naturelle purifiée) pour dissocier du cartilage. La présente invention montre désormais que des collagénases recombinantes peuvent être mises en oeuvre, et que leur utilisation offre de multiples avantages.

Ainsi, dans un mode préféré de mise en oeuvre du procédé de l'invention, dans l'étape a), l'échantillon biologique est traité en présence d'une collagénase recombinante.

Le terme recombinant indique que la collagénase a été produite dans un système artificiel, par expression d'un acide nucléique codant pour cette enzyme. Plus généralement, il s'agit d'une collagénase produite par une cellule hôte dans laquelle un acide nucléique codant une collagénase (ou une fragment ou dérivé fonctionnel de celle-ci) a été introduit artificiellement, ou par une cellule descendant d'une telle cellule (par divisions et/ou dérivations). Par rapport à l'enzyme d'extraction, l'enzyme recombinante présente donc généralement les caractéristiques suivantes :
- structure déterminée
- glycosylations déterminées
- absence de contaminants non identifiés ou pathogènes
- absence de trypsine
- composition connue et homogène.

La collagénase recombinante utilisée est préférentiellement d'origine bactérienne, comme par exemple la collagénase A, la collagénase P, la collagénase de Vibrio alginolyticus (EP430635) ou encore la collagénase ColH de Clostridium histolyticus (J. Bact. 181 (1999) 2816). D'autres collagénases peuvent également être utilisées, comme des collagénases d'origine eucaryote, notamment mammifère, par exemple humaine. En particulier, on peut utiliser des collagénases (recombinantes) de mammifère, spécifiques du collagène de type II et/ou du cartilage ou également des collagénases modifiées, par exemple par mutagénèse dirigée, possédant des propriétés améliorées en terme d'efficacité, de sélectivité, de conditions d'activité, etc. En particulier, il peut s'agir de collagénases modifiées (par exemple par mutagénèse dirigée), capables d'exercer une activité à des températures basses, notamment inférieures à 20°C, plus préférentiellement inférieures à 10°C.

L'utilisation d'une collagénase recombinante selon la présente invention offre de nombreux avantages par rapports aux techniques antérieures, bases sur l'emploi de collagénases naturelles. Le premier avantage réside dans l'absence de contaminants, susceptibles d'affecter la qualité de la préparation de chondrocytes. En effet, la collagénase recombinante selon l'invention est avantageusement produite en conditions de bonnes pratique de fabrication (BPF), c'est-à-dire essentiellement dépourvue de tout contaminant biologique incompatible avec un usage thérapeutique. Le procédé de l'invention permet donc de fournir des préparations de chondrocytes de qualité pharmaceutique. D'autre part, un autre avantage de l'utilisation d'une collagénase recombinante réside dans l'efficacité supérieure de l'enzyme recombinante par rapport à l'enzyme naturelle. Ainsi, les résultats obtenus montrent que la collagénase recombinante permet une dissociation plus rapide et/ou à des doses inférieures de l'échantillon biologique. Ceci constitue un avantage précieux du procédé de l'invention par rapport aux procédés utilisant une collagénase d'extraction.

Ces collagénases peuvent être produites dans tout hôte cellulaire approprié, c'est-à-dire en particulier dans tout hôte cellulaire n'ayant pas de caractère pathogène reconnu.

L'étape de dissociation est généralement réalisée à une température proche de la température du corps humain, par exemple entre 32 et 40°C, de préférence proche de 37°C, pendant une période pouvant être adaptée par l'homme du métier en fonction de la quantité d'enzyme utilisée (de 0,05 à 5 mg/ml, plus préférentiellement de 0,1 à 1 ml/ml environ).

Une étape typique de dissociation selon l'invention comprend la mise en contact de l'échantillon biologique (le cas échéant traité mécaniquement comme décrit ci avant), avec la collagénase recombinante (0,5 mg/ml) pendant environ 15 heures à 37°C, sous agitation.

Pour l'étape de dissociation, la collagénase recombinante peut par ailleurs être utilisée en combinaison avec d'autres enzymes, recombinantes ou d'extraction, dont la qualité aura été contrôlée.

En outre, comme indiqué avant, l'étape de dissociation peut être réalisée dans un milieu de culture nutritif dans lequel l'échantillon biologique est conservé. Dans ces conditions, selon un mode particulier de mise en oeuvre de l'invention, l'échantillon biologique prélevé est placé dans un milieu nutritif (de préférence à basse température, par exemple entre 2 et 8°C environ) comprenant une enzyme capable de dégrader un ou plusieurs constituants de la matrice extracellulaire cartilagineuse, pendant une période pouvant aller de 1 à 15 semaines.

### Etape b) : Cultures monocouches

Les chondrocytes ainsi dissociés de l'échantillon biologique sont, dans une étape b), cultivés en monocouche afin de les multiplier (ou expandre).

Préalablement à la culture, le milieu de digestion (utilisé dans l'étape a)) est éliminé par centrifugation, et les cellules sont lavées plusieurs fois en milieu de culture frais. Les cultures peuvent être réalisées dans tout milieu de culture approprié à la prolifération des cellules mammifère, notamment des cellules humaines. De préférence, des milieux complets sont utilisés dans cette étape, c'est-à-dire des milieux de culture supplémentés de sérum de veau foetal ou de sérum autologue ou de sérum humain AB sécurisé, d'antibiotiques (par exemple gentamycine), de vitamines (par exemple acide ascorbique) et de HEPES. Des milieux particuliers utilisables sont par exemple les milieux HAM F12, DMEM et RPMI, seuls ou en combinaison(s), le cas échéant supplémentés. Des milieux spécifiques ont été décrits dans la littérature, qu'il s'agisse de milieux définis, de milieux sans sérum etc. (voir notamment Adolphe et al., Exp. Cell. Res. 155 (1984) 527 ; Jennings et al., Cell Biol. Int. Reports 7 (1983) 149 ; WO98/04681, incorporés à la présente par référence). De manière typique, les cellules sont ensemencées à une concentration initiale inférieure à 10 000 cellules /cm2, généralement entre 5 000 et 10 000 cellules. Le milieu est remplacé généralement tous les 2 à 3 jours par du milieu frais.

La culture monocouche peut être réalisée dans différents supports de culture appropriés, tels que des boites, flasques, poches, bouteilles roulantes, appareils « multi tray », « cellcube », cartouches, etc. A cet égard, selon une variante particulière de l'invention, les cultures monocouches sont réalisées dans des isolateurs portables ou des bouteilles de section triangulaire, munis de pertuis et de chambres de culture de tailles différentes. Ce type de dispositif permet avantageusement d'effectuer plusieurs passages, dans des dispositifs stériles et en contrôlant aisément l'apport et les remplacements des milieux de culture.

Les cultures monocouches peuvent être réalisées pendant une durée variable . Généralement, elles sont réalisées jusqu'à ce que les cellules approchent de la confluence, soit de 1 à 4 semaines, plus souvent de 2 à 4 semaines, généralement de l'ordre de 3 semaines.

### Etape c) : Détachement des cellules

Les chondrocytes en culture, notamment en culture monocouche, adhèrent aux parois du support utilisé pour la culture. Il convient donc, pour les récolter, de les détacher des parois.

Généralement, les chondrocytes sont détachés du support lorsque la culture approche de la confluence. Plus précisément, l'étape de détachement des cellules est réalisée lorsque la culture atteint environ 60 à 95% de la confluence, plus préférentiellement 60 à 90% de la confluence. Ce stade peut être mis en évidence par l'homme du métier par les techniques conventionnelles (mesure de densité optique, examen visuel, etc.). Pour la mise en oeuvre de cette étape, le milieu de culture est préférentiellement éliminé, puis les cellules sont incubées dans un milieu frais comprenant le ou les composés choisis (milieu de détachement). Il est entendu que les composés peuvent être ajoutés directement au milieu de culture des cellules, bien que ce mode de réalisation ne soit pas préféré. Le milieu utilisé pour le détachement peut être de composition identique à celui utilisé pour la culture monocouche des cellules. Il peut bien entendu s'agir également de tout autre milieu adapté à la culture de cellules mammifère, notamment des chondrocytes. Préférentiellement, il s'agit d'une solution saline tamponnée comprenant le ou les composés actifs (enzymes, chélateurs, etc.). Dans ce cas, dès que les composés ont agi (généralement moins de 10 minutes après leur incubation avec les cellules), du milieu de culture complet est ajouté afin d'inhiber leur action et de protéger les cellules. Si nécessaire, la monocouche cellulaire (plage) peut alors être aisément dissociée mécaniquement (pipette).

Comme indiqué, selon un mode de réalisation avantageux de la présente invention, les chondrocytes sont détachés par incubation en présence d'un milieu comprenant au moins un composé choisi parmi les dérivés polyosidique.

Le dérivé polyosidique peut être de nature différente. Il s'agit plus généralement d'un polymère poyosidique, d'origine naturelle ou synthétique, mais compatible avec un usage clinique. Avantageusement, le polymère polyosidique est un polysacchararide, notamment un polysaccharide sulfaté, ayant de préférence un poids moléculaire moyen compris entre 5 000 et 500 000 dalton, plus préférentiellement entre 5 000 et 100 000 daltons. Le polysaccharide sulfaté peut être choisi de préférence parmi l'héparine et le dextran-sulfate, de manière tout particulièrement préférée, l'héparine.

L'héparine a un poids moléculaire moyen de 20 000 Da environ, et est utilisée comme médicament anti-coagulant, ainsi que des dérivés de plus petit poids moléculaire de celle-ci. Par exemple, l'héparine est commercialisée, sous forme lyophilisée, par la société Choay.

Pour une bonne utilisation dans le procédé de l'invention, le dérivé polyosidique est préférentiellement ajouté à une concentration comprise entre environ 30 et environ 2000 unités par ml, avantageusement entre environ 50 et environ 1000 Ul/ml, encore plus préférentiellement entre environ 100 et environ 1000 U/ml. Par ailleurs, le milieu de détachement peut comprend, outre le dérivé polyosidique, des chélateurs, tels que l'EDTA par exemple.

### Etape d) : Expansion des chondrocytes

Dans le procédé de production de l'invention, afin de multiplier la quantité de chondrocytes, plusieurs passages en culture monocouche peuvent être accomplis.
Comme indiqué précédemment, la répétition des étapes b) et c) du procédé, et l'expansion des chondrocytes qui en résulte, sont particulièrement importantes pour des application de greffes autologues, où il est essentiel de produire une quantité suffisante de chondrocytes, à partir d'une biopsie, pour pallier au défaut cartilagineux du patient.

Généralement, les étapes b) et c) peuvent être répétées de une à dix fois, sans nuire à la viabilité cellulaire. La réalisation de 10 passages permet la plupart du temps de générer un nombre satisfaisant de chondrocytes pour toute application thérapeutique, à partir de tout échantillon biologique. Plus souvent, le procédé de l'invention comprend de 1 à 7 passages des cellules, par exemple 3. Il est entendu que le nombre de passages peut être aisément adapté par l'homme du métier, en fonction du volume initial de l'échantillon biologique, des besoins, etc.

### Récupération et conditionnement des chondrocytes

Les cellules ainsi produites peuvent être récupérées et conditionnées pour des usages multiples. En général, les cellules issues d'un même échantillon biologique sont collectées et assemblées, ce qui permet d'obtenir des compositions de cellules autologues.

Ces cellules peuvent tout d'abord être remises en suspension dans tout milieu désiré et utilisées telles quelles, comme produit thérapeutique. En effet, l'implantation de suspensions de chondrocytes, notamment autologues, pour des applications cliniques dans la régénération du tissu cartilagineux à été rapportée dans la littérature. A cet égard, bien que les chondrocytes se dédifférencient généralement au cours des différents passages, il a été montré que des suspensions de chondrocytes pouvaient se redifférencier in situ, après implantation. Il est donc possible de préparer des compositions comprenant des suspensions de chondrocytes obtenus selon le procédé ci-dessus, par exemple de 10⁵ à 10⁸ chondrocytes par dose. Dans un mode particulier de réalisation, les chondrocytes sont conditionnés dans un milieu gélifié, assurant une plus grande maniabilité et une meilleure prise de l'implant.

Dans ce but, les cellules récoltées peuvent être lavées plusieurs fois dans du milieu de culture frais, puis reprises dans toute solution physiologique (tampon, saline, etc.) adaptée à un usage thérapeutique, et notamment à une administration in vivo. Typiquement, les cellules peuvent être reprises dans une solution d'albumine à 4% ou dans du sérum autologue ou du sérum humain AB sécurisé, dans un volume choisi pour obtenir la concentration de cellules voulue. A titre d'exemple, les cellules reprises dans un volume de 0,4 ml permettent d'obtenir une concentration de cellules autologues supérieure à 10⁷ cellules/ml, par exemple. Ces cellules peuvent être placées dans des tubes, ampoules, seringues, etc., en conditions stériles, et utilisées pour l'injection.

Les chondrocytes obtenus (après un ou plusieurs passages), peuvent également être mis en culture dans un milieu gélifié, afin de les expandre et de faciliter leur implantation ultérieure.

Dans une autre variante, les chondrocytes récoltés (après un ou plusieurs passages) peuvent être remis en culture, dans certaines conditions, afin de favoriser leur re-différenciation. Ceci permet de produire in vitro des chondrocytes sécrétant une matrice extracellulaire (notamment du collagène de type II et des protéoglycans).

Cette nouvelle culture peut s'effectuer par exemple en culture tridimensionnelle (par exemple en présence d'une matrice biocompatible), ou dans des supports non-adhérents.

La culture tridimensionnelle, par exemple en billes d'alginate ou sur des fibres biocompatibles a été décrite dans la littérature (Guo et al., Conn. Tiss. Res. 19 (1989) 277; US5,736,372 ; US5,041,138 ; US4,846,835 ; US4,642,120). Ces techniques permettent de former in vitro, des matrices prêtes à l'implantation dans les zones à traiter.

La culture dans des supports non-adhérents a été décrite par exemple dans la demande WO95/30742. Il s'agit essentiellement de cultiver les cellules dans des support dont la surface a été fonctionnalisée pour prévenir l'adhésion cellulaire. Les cellules sont ainsi capables de proliférer en suspension, de se redifférencier, et de produire une matrice extracellulaire endogène dans laquelle elles se trouvent incluses. Cette approche conduit à la constitution, in vitro, de cartilages artificiels, de volume et forme prédéterminées, utilisable pour l'implantation.

Les chondrocytes produits peuvent également être manipulés, par exemple pour y introduire des acides nucléiques hétérologues, en vue de leur conférer des propriétés supplémentaires.

Les chondrocytes peuvent aussi être utilisés in vitro, en culture ou en suspension, pour des études sur la différenciation cellulaire, la recherche de gènes thérapeutiques, la purification de protéines, etc.

### Congélation des chondrocytes

La présente invention décrit également des compositions et méthodes particulièrement efficaces pour conserver les chondrocytes, en particulier sous forme congelée. Ces méthodes permettent avantageusement de conserver les chondrocytes sur de longues périodes, sans affecter leurs propriétés fonctionnelles. En outre, des compositions et méthodes particulières utilisées sont compatibles avec un usage thérapeutique, et permettent donc de préserver les chondrocytes ou les compositions de chondrocytes destinées à être implantées in vivo.

La congélation peut être réalisée dans différentes conditions, et en présence de différents agents ou compositions protecteurs.

Selon une première variante de réalisation, la congélation est réalisée en présence de diméthyl sulfoxyde (« DMSO »). Le DMSO est un agent protecteur bien connu, qui s'insère dans les membranes cellulaires et permet de les stabiliser, ce qui empêche la destruction des cellules. Un milieu de congélation typique au DMSO comprend par exemple de 5 à 25 % de DMSO et du sérum de veau foetal. Toutefois, dans la mesure où le DMSO ne peut être injecté à l'homme, ce type de milieu n'est pas tout à fait adapté à un usage thérapeutique. En effet, il nécessite au moins une étape de traitement des cellules après décongélation (centrifugation, rinçages, etc.) afin d'éliminer le DMSO, préalablement à toute administration à un sujet. Ce type de congélation peut toutefois être utilisé pour d'autres utilisations des chondrocytes, telles que mentionnées ci avant.

De manière particulièrement avantageuse, la congélation est cependant réalisée en absence de DMSO. Plus particulièrement, la présente invention décrit à présent des méthodes et compositions pour la congélation des chondrocytes en conditions compatibles avec un usage thérapeutique direct (i.e., sans traitement des compositions décongelées). A cet égard, dans un mode de réalisation préféré de l'invention, la conservation des chondrocytes, notamment sous forme congelée, est effectuée dans un milieu dépourvu de DMSO, comprenant de la sérum albumine (humaine), une solution saline, et une gélatine modifiée. Un telle composition a été décrite dans la demande FR 2,746,109. Dans un autre mode préféré de mise en oeuvre, la congélation est réalisée dans un milieu comprenant de l'albumine (humaine), un polysaccharide, et éventuellement une solution saline. La présente demande montre en effet que ce type de milieu peut être utilisé pour la congélation de chondrocytes. L'invention décrit également les conditions de préparation des chondrocytes pour améliorer leur conservation.

Un objet particulier de l'invention réside donc dans une composition comprenant des chondrocytes, notamment des chondrocytes humains, une solution saline, de l'albumine humaine et une gélatine modifiée ou un polysaccharide, de préférence un polysaccharide. Encore plus préférentiellement, de telles compositions selon l'invention comprennent au moins environ 5.10⁶ chondrocytes/ml. En effet, la présente invention montre que les cellules peuvent être conservées de manière plus efficace (notamment en assurant une meilleure viabilité à la décongélation), lorsqu'elles sont congelées à une concentration de au moins environ 5.10⁶ chondrocytes/ml, encore plus préférentiellement de 10⁷ chondrocytes/ml, environ, ou plus.

Le milieu de conservation de l'invention est généralement préparé en mélangeant les différents constituants entre eux, puis en ajoutant ledit milieu aux chondrocytes, de préférence à une préparation de chondrocytes à une concentration de l'ordre de 10⁷ cellules/ml environ. Les chondrocytes peuvent être des chondrocytes primaires, fraichement isolés à partir d'un échantillon biologique, ou bien des chondrocytes multipliés in vitro (par exemple par cultures monocouches), ou bien encore des chondrocytes redifférenciés, par culture tridimensionnelle (par exemple en billes d'alginate).

Pour la congélation des chondrocytes, la solution saline utilisée peut être plus particulièrement une solution isotonique avec le plasma. Les sels entrant dans la composition de cette solution peuvent varier. Avantageusement elle comprend des chlorures, tels que du chlorure de sodium, du chlorure de potassium, du chlorure de calcium et/ou du chlorure de magnésium, et des lactates, tels que par exemple du lactate de sodium. Dans un exemple typique, la solution saline isotonique comprend du chlorure de sodium, du chlorure de potassium, du chlorure de magnésium et du lactate de sodium. Selon une autre variante, le chlorure de magnésium est remplacé par du chlorure de calcium. Dans ce cas les concentrations en sels de la solution saline sont équivalentes ou quasi équivalentes à celles d'une solution « Ringer-lactate ». Une telle solution est habituellement utilisée en perfusion pour compenser une déshydratation ou une perte de liquide physiologique par exemple.

Selon un mode particulier de réalisation de l'invention, la solution saline est composée essentiellement de NaCI MgCl2, KCl et lactate dans des gammes de concentrations comprises respectivement entre 2 et 9 g/l ; 0,05 et 0,2 g/l ; 0,05 et 0,5 g/l et 0,5 à 5 g/l.

Les dérivés de gélatine utilisés pour la conservation des chondrocytes sont plus particulièrement des gélatines fluides modifiées. De telles gélatines fluides modifiées selon l'invention sont typiquement constituées de produits d'hydrolyse du collagène modifiés chimiquement, compatibles avec une utilisation pharmaceutique. Il s'agit préférentiellement de produits ayant un poids moléculaire moyen compris entre 10 kD et 100 kD, et encore plus préférentiellement entre 15 kD et 40 kD. Ils sont préférentiellement modifiés par réaction avec un anhydride, de manière à obtenir un produit final ayant une fluidité adaptée a l'usage recherché, selon les enseignements par exemple du brevet FR 1,291,502. Il s'agit préférentiellement de l'anhydride succinique, citraconique, itaconique, aconitique ou maléique. Une gélatine fluide modifiée particulièrement avantageuse est constituée du produit d'hydrolyse du collagène ayant un poids moléculaire moyen compris entre 15 kD et 40 kD, modifié par réaction avec l'anhydride succinique. Les gélatines fluides modifiées selon l'invention peuvent être préparées par les techniques de l'homme de l'art. Parmi les gélatines fluides modifiées, on peut citer à titre d'exemple l'oxypolygélatine, obtenue par polymérisation de la gélatine avec le glyoxal et oxydation par H202. D'autres gélatines fluides modifiées sont obtenues par réaction de la gélatine (ayant de préférence une gamme de poids moléculaire d'environ 15.000 à 36.000) avec l'anhydride succinique, citraconique, itaconique, aconitique ou maléique ou le chlorure de succinyle ou de fumaryle, comme décrit dans le brevet français n° FR1,291,502. Tous ces dérivés de gélatine sont compatibles avec une utilisation pharmaceutique et peuvent être introduits dans le courant sanguin directement en solution saline isotonique. Des gélatines fluides modifiées ont également été décrites dans les brevets US2,525,753, US2,827,419, US3,108,995.

La sérum albumine utilisée est une sérum albumine humaine (SAH), d'extraction ou recombinante. La SAH naturelle d'extraction peut être produite par purification à partir de matériel biologique d'origine humaine, par les techniques classiques de fractionnement du plasma provenant de dons de sang (Cohn et al., J. Am. Chem. Soc. 68 (1946) 459 pp), ou par extraction à partir du placenta humain, selon la technique décrite par J. Liautaud et al. (13ème Congrès International d'ABS, Budapest; A: "Purification of proteins. Development of biological standard", Karger (ed.), Bale, 27 (1973) 107 pp). De préférence l'albumine purifiée utilisée dans le cadre de la présente invention est une albumine plasmatique. Tout particulièrement on peut utiliser une solution d'albumine plasmatique commerciale. La SAH recombinante peut être fabriquée dans différents types d'hôtes cellulaires, de préférence eucaryote (Cf FR 2,746,109).

Dans les milieux de conservation, le polysaccharide utilisé peut être de structure et de poids moléculaire variable. Préférentiellement, il s'agit d'un polysaccharide sulfaté, ayant de préférence un poids moléculaire compris entre 5000 et 500 000 dalton, plus préférentiellement entre 30 000 et 250 000 daltons. Le polysaccharide peut être choisi par exemple parmi le dextran (40 000 ou 60 000 daltons), l'amidon, l'hydroxyéthylamidon (240 000 dalton), etc.

Dans un mode particulier de mise en oeuvre, le milieu de l'invention est composé de plasmion (FR 2,042,381) auquel de la sérum albumine humaine est ajoutée, à des concentrations variables.

Dans un autre mode, préféré, de mise en oeuvre, le milieu de l'invention est composé de Rhéomacrodex^{R}, Hémodex^{R}, Plasmaclair^{R} ou Hestéril^{R}, auquel de la sérum albumine humaine est ajoutée, à des concentrations variables (de 5 à 45% pour une solution d'albumine à 20%).

Si nécessaire, les concentrations respectives des différents constituants peuvent être ajustées en utilisant la méthodologie suivante :
Sur des plaques multi-puits, sont répartis, dans chaque puits, chacun des constituants du milieu à une concentration fixe, à l'exception d'un constituant dont on fait varier la concentration. Le cas échéant, plusieurs plaques sont préparées, permettant de tester simultanément des conditions de concentrations différentes pour chacun des constituants, ou, si le nombre de puits est suffisant, ces différentes conditions sont testées sur une même plaque. Préférentiellement, chaque condition est testée au moins en double, de préférence en triple sur la plaque. Une préparation de chondrocytes est alors introduite dans chaque puits, à une concentration de l'ordre de 10⁷ cellules /ml. La plaque est placée dans l'azote liquide à -80°C (éventuellement après une étape intermédiaire au congélateur). Les plaques congelées sont ensuite décongelées, et la viabilité cellulaire est déterminée dans chaque puits, par exemple par coloration au bleu trypan. Chaque plaque peut être lue au moyen d'un système robotisé, de manière à permettre de traiter de nombreuses conditions et d'analyser aisément les résultats obtenus.

Les composition de chondrocytes selon l'invention peuvent être conservées sous forme congelée, par exemple à -80°C, sans affecter significativement les propriétés fonctionnelles des cellules. Comme indiqué dans les exemples, une viabilité cellulaire supérieure à 85% peut être obtenue.

### Utilisations pour la greffe de chondrocytes

L'invention a également pour objet l'utilisation de chondrocytes produits par le procédé décrit ci avant ou d'une composition décrite ci avant, pour la préparation d'une composition destinée à l'implantation de chondrocytes chez l'homme, en particulier pour traiter des défauts cartilagineux. Le terme « traiter des défauts cartilagineux » désigne notamment la restauration ou la compensation de défauts cartilagineux, c'est à dire en particulier la reconstitution, au moins partielle, de cartilage dans des zones ou celui-ci est défectueux. De préférence, il s'agit d'une utilisation dans un contexte autologue, c'est-à-dire que l'échantillon biologique utilisé pour produire les chondrocytes provient du sujet auquel les chondrocytes produits seront administrés.

L'invention concerne également une méthode pour restaurer des cartilages in vivo, comprenant l'administration à un sujet d'une composition de chondrocytes produite selon le procédé de l'invention, de préférence de chondrocytes autologues.

A cet effet, la méthode de l'invention comprend avantageusement le prélèvement d'un échantillon de cartilage sain chez le sujet, la préparation de chondrocytes à partir de cet échantillon, selon le procédé décrit avant, puis l'administration, à ce sujet, d'une composition de chondrocytes ainsi obtenus.

L'invention concerne également une méthode pour le traitement de pathologies du cartilage, comprenant l'administration à un sujet d'une composition de chondrocytes produite selon le procédé de l'invention, de préférence de chondrocytes autologues.

Les méthodes et compositions de l'invention peuvent également être mises en oeuvre avec des chondrocytes allogéniques, voire xénogéniques. Ainsi, l'invention propose pour la première fois la réalisation de banques de chondrocytes, en particulier de banques de chondrocytes allogéniques, utilisables pour le traitement ou la réparation de défauts cartilagineux. L'utilisation de chondrocytes allogéniques peut être avantageuse puisqu'elle permet l'implantation en un seul temps opératoire, sans nécessiter de préparation patient par patient. Cette nouvelle approche est donc économiquement très avantageuse, efficace sur le plan biologique et thérapeutique, et intéressante en raison de la faible vascularisation des cartilages, limitant fortement les risques de rejet.

Un objet de l'invention réside donc dans l'utilisation de chondrocytes allogéniques ou xénogéniques pour le traitement de défauts cartilagineux. L'invention réside en particulier dans une méthode pour le traitement de pathologies du cartilage, comprenant l'administration à un sujet d'une composition de chondrocytes allogéniques ou xénogéniques, de préférence produite selon le procédé de l'invention. Les chondrocytes allogéniques ou xénogéniques peuvent être congelés dans les conditions décrites ci-avant. En outre, ils peuvent être éventuellement sélectionnés ou traités pour exprimer ou porter certains déterminants HLA. L'invention concerne également une banque de chondrocytes humains allogéniques, ou éventuellement xénogéniques, de préférence sous forme congelée, encore plus préférentiellement présentant des déterminants HLA sélectionnés, en vue de leur utilisation pour la réparation ou le traitement de défauts du cartilage chez des sujets humains.

Avant l'implantation de la composition de chondrocytes, il est préférable de préparer la zone à traiter. A cet effet, le défaut peut être préparé pour assurer une meilleure prise de l'implant (notamment son attachement), pour réduire les risques de vascularisation, etc. Généralement, le défaut est préalablement curé (afin d'éliminer de la zone tout le cartilage défectueux), puis nettoyé. Ensuite, différentes techniques d'implantation peuvent être mises en oeuvre, selon le matériel implanté (suspension, matrice, cartilage reconstitué in vitro).

En particulier, l'implantation peut être effectuée selon la technique décrite dans la demande W098/08469, impliquant le dépôt, sur le site à traiter, d'une couche de collagène pour éviter ou réduire la vascularisation, puis, sur cette première couche, des chondrocytes, puis, sur les chondrocytes, d'un patch constitué de collagène semi-perméable.

L'implantation peut également être réalisée selon la technique décrite dans US4,846,835, consistant à accrocher mécaniquement l'implant au moyen d'un flap périostal suturé au cartilage.

D'une manière générale, l'implantation peut être réalisée par différentes techniques chirurgicales connues de l'homme du métier, et éprouvées en clinique humaine, impliquant notamment une étape de fixation de l'implant pendant la chirurgie, par des sutures biodégradables ou par application de bioadhésifs (voir par exemple Minas et al., Operative Techniques in Orthopaedics 7 (1997) 323, incorporé à la présente par référence). Des exemples de bioadhésifs sont notamment des colles biologiques à la fibrine et/ou à la thrombine (FR2,448,900) ou d'autres matières biocompatibles telles que Tissucol et Vieryl (voir également US5,197,973).

A cet égard, la présente invention décrit une nouvelle méthode pour l'implantation de chondrocytes in vivo, comprenant la fixation, sur la zone à traiter, d'un film biocompatible (préférentiellement résorbable (biodégradable)) contenant ou destiné à recevoir les chondrocytes multipliés in vitro. Plus particulièrement, le film biocompatible résorbable est fixé sur la zone à traiter au moyen d'une colle biologique ou biocompatible. Dans une variante préférée, le film est positionné sur le défaut cartilagineux, puis la poche ainsi constituée est remplie de chondrocytes multipliés in vitro. Ce mode d'implantation selon l'invention est avantageux dans la mesure où il évite le recours à des sutures périostales. Dans un mode plus préféré de réalisation, l'implantation est effectuée par application, au moyen d'une colle biologique, d'un film biocompatible résorbable sur le site à traiter, puis une composition de chondrocytes en solution liquide ou gélifiée est introduite dans la poche formée par le film.

L'invention réside donc également dans l'utilisation d'un film biocompatible, en particulier résorbable, pour la préparation d'une composition destinée à l'implantation de chondrocytes sur un cartilage défectueux. Il s'agit plus préférentiellement de chondrocytes en solution liquide ou gélifiée, multipliés in vitro. Encore plus préférentiellement, il s'agit de chondrocytes autologues.

Les compositions et méthodes de l'invention peuvent être utilisées pour traiter tout défaut du cartilage, notamment au niveau du fémur, du tibia, du ménisque, des côtes, etc.

D'autres aspects et avantages de la présente invention apparaîtront à la lecture des exemples qui suivent, qui doivent être considérés comme illustratifs et non limitatifs.

### Exemples spécifiques

### Exemple 1 : Mise au point de milieux de détachement des chondrocytes en culture

Cet exemple décrit la mise au point de nouveaux milieux de détachement de chondrocytes en culture. Ces milieux présentent l'avantage de détacher les chondrocytes sans altérer de manière significative les membranes cellulaires ou les propriétés biologiques des cellules.

Après digestion enzymatique pendant 12 heures à 37°C, le cartilage de lapin a été rincé trois fois avec du milieu F-12 Ham, puis les cellules ont été mises en culture dans une flasque avec 15 ml de milieu complet (HamF12, 10%SVF, 1%hépès, 1% antibiotiques et 50pg/ml de vitamine C. Après confluence, les cellules ont été repiquées avec un milieu trypsine-EDTA et mises en culture dans des plaques 24 puits pendant 4 à 5 jours. Deux concentrations cellulaires ont été testées (2.10⁴ et 5.10⁴ cellules/puits). Différents milieux de détachement des cellules ont ensuite été testés, à raison de 500 µl/puits. Les résultats obtenus sont présentés dans le Tableau 1.

Ils montrent que les solutions testées ont permis de détacher les cellules du support, pour produire des cellules isolées (dissociées les unes des autres) ou des plages de cellules détachées du support. Les résultats obtenus montrent notamment que les milieux de l'invention utilisant des dérivés polyosidiques, en particulier de l'héparine, le cas échéant en association avec l'EDTA, permettent de détacher efficacement les cellules, y compris à des concentrations faibles (100Ul/ml notamment). Les résultats obtenus montrent par ailleurs que les cellules ainsi obtenues sont capables de proliférer de manière normale, après remise en culture.

### Exemple 2 : Production et congélation de chondrocytes

Des chondrocytes articulaires humains sont isolés à partir d'un échantillon de cartilage, par traitement mécanique puis enzymatique. A cet effet, une biopsie, prélevée au bloc opératoire est placée stérilement dans un tube de 50 ml contenant un milieu nutritif (HAM F12 supplémenté de 50 µg/ml de gentamycine). Après vérification de l'absence de contamination, la biopsie est découpée à l'aide d'une pince et d'un scalper stérile en morceaux de dimension inférieure à 3 mm³ environ. Ces morceaux sont ensuite incubés, dans un tube, en présence de milieu HAM F12 contenant 1mg/ml de collagénase A, d'extraction ou recombinante. Les chondrocytes articulaires humains obtenus sont lavés plusieurs fois puis mis en culture en monocouche pendant 21 jours. Après cette période, les cellules sont décollées en présence d'une solution enzymatique trypsine (0,005%) - EDTA (0,002%) ou d'une solution d'héparine, puis lavées deux fois dans du milieu de culture complet. Les cellules ainsi détachées sont récoltées et remises en suspension délicatement à différentes concentration à 4°C, d'une part dans un milieu de congélation comprenant du DMSO, d'autre part dans des milieux de congélation dépourvus de DMSO et de glycérol, de composition suivante :
Milieu 1 :
   - SAH à 20% : 33%
   - Rhéomacrodex^{R} : 67%
Milieu 2 :
   - SAH à 20% : 33%
   - Hémodex^{R} : 67%
Milieu 3
   - SAH à 20% : 33%
   - Plasmadair^{R} : 67%
Milieu 4 :
   - SAH à 20% : 33%
   - Hestéril^{R} : 67%

Les composition obtenues sont déposées à -80°C puis conservées dans l'azote liquide. Les chondrocytes sont ensuite décongelés au bain-marie à 37°C, pour analyse.

Une mesure de la viabilité cellulaire a ensuite été réalisée à différents temps, à l'aide d'une coloration vitale d'exclusion au bleu trypan. Les résultats obtenus pour le milieu de congélation 1 (sans DMSO) sont présentés dans le Tableau 2. Ils montrent qu'une très bonne viabilité cellulaire est obtenue (jusqu'à 87% de viabilité), notamment lorsque les cellules sont congelées à des concentrations élevées (de l'ordre de 10⁷ cellules par ml de milieu). Des résultats similaires sont obtenus avec les milieux 2-4 définis ci-dessus. Ces résultats montrent donc que les chondrocytes peuvent être congelés, en l'absence de DMSO, avec un pourcentage de viabilité très élevé, notamment lorsqu'ils sont congelés à une concentration de 10⁷ cellules par ml de milieu environ, ou plus.

### Exemple 3 : Analyse du phénotype

Cet exemple montre que les chondrocytes produits selon l'invention, en particulier les chondrocytes décongelés, conservent leurs propriétés fonctionnelles et sont capables de se différencier pour produire une matrice extracellulaire cartilagineuse.

Les chondrocytes articulaires cultivés en monocouche perdent leur caractère différencié au cours des passages successifs. Ce processus conduit notamment à une diminution progressive de la synthèse de collagène de type II, considéré comme le marqueur spécifique du cartilage, et l'apparition de collagène de type I et III, non caractéristique de ce tissu. Cet exemple montre que les chondrocytes ainsi dédifférenciés peuvent retrouver leur phénotype primaire lorsqu'ils sont transférés de la culture monocouche à la culture tridimensionnelle (en agarose, en alginate) ou implantés in vivo.

Afin de tester les potentialités des chondrocytes dédifférenciés, amplifiés en monocouche et destinés à être réimplantés, l'expression du collagène de type II a été mesurée par RT-PCR, sur des chondrocytes multipliés in vitro, congelés, décongelés, puis transférés en culture tridimensionnelle.

### 3.1. Cultures cellulaires

Dans ce but, des chondrocytes produits et congelés selon l'exemple 1 ou 2 ont été décongelés puis transférés en billes d'alginate (Guo et al., Comm. Tissue Res. 19 (1989) 277). Brièvement, les cellules ont été mises en suspension dans une solution d'alginate (1,42%) de faible viscosité, stérilisée par autoclavage, à la densité de 1,5.10⁶ cellules/ml. La suspension a ensuite été versée goutte à goutte au moyen d'une seringue équipée d'une aiguille de « 22-gauge », dans une solution de CaCl₂ 100 mM. Après gélification des billes, celles ci ont été laissées pendant 10 min dans la solution afin de terminer leur polymérisation. Elles ont ensuite été rincées 3 fois dans l'EBSS (Earle's buffered saline solution) puis 1 fois dans du DMEM. Enfin, elles ont été placées dans du DMEM + 10% SVF et cultivées pendant 13 jours à 37°C, 5% CO₂, avec changement de milieu tous les 3 jours.

### 3.2. Extraction de l'ARN total

Les billes ont été rincées 3 fois par du PBS à 4°C, puis 4 volumes de tampon de dissolution (citrate de sodium 55mM/EDTA 25 mM) sont ajoutés sur les billes rassemblées au fond d'un tube de 50 ml. La suspension est incubée à 37°C, sous agitation douce, jusqu'à ce que les billes soient complètement dissociées (environ 10 min). Une centrifugation à 800g pendant 10 min permet ensuite de séparer les chondrocytes de la matrice alginate. Le culot de cellules a été soumis à une extraction par l'isothiocyanate de guanidium pour obtenir l'ARN total.

### 3.3. Reverse Transcription -Polymerase Chain Reaction (RT-PCR)

Une transcription inverse a été réalisée, dans un volume de 20 µl contenant 1 µl d'oligo-d(T)₁₆ (20µM), 1 µl de Rnase out (Recombinant Ribonuclease Inhibitor, 40U/µl), 4 µl de tampon « first strand 5X », 1 µl de tampon dNTPs (10 mM) et 0,4 µl de reverse transcriptase (MMLV, 200 U/µl). L'échantillon a été incubé 15 min à 42°C, 5 min à 99°C, puis 5 min dans la glace. 5 µl du produit de RT ont été utilisés pour la PCR en présence des amorces spécifiques pour la b-actine, le collagène de type II (COL2A1) et le collagène de type I (COL1A1). La PCR est réalisée dans un volume final de 20 µl en présence de dNTPs, des amorces et de l'ADN polymérase Taq. Les produits de PCR ont été séparés sur gel d'agarose 1,5% en présence de bromure d'éthydium puis visualisés sous UV.

### 3.4. Résultats

Les résultats obtenus montrent la présence d'ARN messager codant pour le collagène II, dès 25 cycles d'amplification. En revanche, l'ARNm du collagène I est indétectable, même après 35 cycles d'amplification. La même analyse conduite sur un extrait provenant de fibroblastes dermiques humains a servi de contrôle. Ce contrôle montre la présence d'ARNm du collagène I sans expression d'ARNm du collagène II. Ces résultats montrent que les cellules de l'invention recouvrent un phénotype chondrocytaire différencié. Ces résultats montrent donc que les étapes de culture in vitro et de congélation n'ont pas affecté le phénotype des cellules.

**Tableau 1**

| Décollement des chondrocytes par différents milieux | | | | |
|---|---|---|---|---|
| Cellules/puits | Milieu de dissociation | Concentration | Durée du traitement | Aspect des cellules |
| 2.10⁴ | Héparine | 1000 Ul/ml (en PBS) | 20 min | Cl |
| | | 500 Ul/ml (en PBS) | 15 min | Cl |
| | | 100 Ul/ml (en PBS) | 15 min | Cl |
| | EDTA/Héparine | 1 :5000/1000 Ul/ml | 15 min | Cl |
| | | 1 :5000/500 Ul/ml | 15 min | Cl |
| | | 1 :5000/100 Ul/ml | 15 min | Cl |
| 5.10⁴ | Héparine | 1000 Ul/ml (en PBS) | 60 min | Cl+P |
| | | 500 Ul/ml (en PBS) | 60 min | Cl+P |
| | | 100 Ul/ml (en PBS) | 60 min | Cl+P |
| | EDTA/Héparine | 1 :5000/1000 Ul/ml | 60 min | Cl+P |
| | | 1 :5000/500 Ul/ml | 60 min | Cl+P |
| | | 1 :5000/100 Ul/ml | 60 min | Cl+P |
| Cl : Cellules Isolées (dissociées les unes des autres) | | | | |
| P : Plages (tapis cellulaires se décollant par plages) | | | | |

**Tableau 2**

| Viabilité des chondrocytes humains après congélation en azote liquide dans un milieu dépourvu de DMSO | | | |
|---|---|---|---|
| # cartilage | Temps de congélation | Concentration cellulaire (cellules/ml) | % de survie à la décongélation |
| 8 | 24h | 10⁷ | 82 |
| 13 | 24h | 10⁷ | 66 |
| 13bis | 24j | 10⁷ | 87 |
| 14 | 16j | 1,1.10⁶ | 24 |

## Revendications

1. Milieu pour la conservation de cellules eucaryotes, comprenant :
- de la sérum-albumine humaine,
- un polysaccharide, et
- éventuellement une solution saline.

2. Milieu selon la revendication 1, caractérisé en ce que les cellules eucaryotes sont des cellules présentatrices d'antigènes, notamment des monocytes, macrophages, cellules dendritiques ou leurs dérivés.

3. Milieu selon la revendication 1, caractérisé en ce que les cellules eucaryotes sont des chondrocytes.

4. Composition pour la congélation de chondrocytes, caractérisée en ce qu'elle est dépourvue de DMSO et/ou de glycérol, et compatible avec un usage thérapeutique.

5. Composition comprenant des chondrocytes, notamment des chondrocytes humains, une solution saline, de l'albumine humaine et un dérivé de gélatine.

6. Composition comprenant des chondrocytes, notamment des chondrocytes humains, de l'albumine humaine, un polysaccharide et, éventuellement, une solution saline.

7. Composition selon la revendication 5 ou 6, caractérisée en ce que l'albumine humaine est une préparation d'albumine d'extraction purifiée.

8. Composition selon la revendication 6, caractérisée en ce que le polysaccharide est un polysaccharide sulfaté ayant un poids moléculaire moyen compris entre 5 000 et 500 000, de préférence entre 30 000 et 250 000.

9. Composition selon l'une des revendications 4 à 8, caractérisée en ce qu'elle comprend au moins environ 5.10⁶ chondrocytes/ml, avantageusement au moins environ 10⁷ chondrocytes/ml.

10. Méthode de production d'une préparation de chondrocytes à partir d'un échantillon biologique, comprenant (a) le traitement de l'échantillon en présence d'une enzyme permettant la dissociation des chondrocytes, (b) la culture en monocouche des chondrocytes ainsi dissociés, (c) la mise en contact des chondrocytes avec un milieu permettant de détacher les chondrocytes adhérents et (d) la congélation de chondrocytes obtenus, caractérisée en ce que la congélation est réalisée dans un milieu dépourvu de DMSO et/ou de glycérol et compatible avec un usage thérapeutique.

11. Méthode selon la revendication 10, caractérisée en ce que les chondrocytes sont congelés dans un milieu comprenant de l'albumine humaine, un polysaccharide et, éventuellement, une solution saline.

12. Méthode selon la revendication 10 ou 11, caractérisée en ce que les cellules sont congelées à une concentration supérieure ou égale à environ 5.10⁶ cellules/ml.

13. Méthode de congélation de chondrocytes, notamment de chondrocytes humains, comprenant la mise en contact d'une préparation de chondrocytes, notamment de chondrocytes humains, avec un milieu comprenant une solution saline, de l'albumine humaine et un dérivé de gélatine, le milieu étant dépourvu de DMSO et/ou de glycérol, et la congélation de la composition obtenue.

14. Méthode de congélation de chondrocytes, notamment de chondrocytes humains, comprenant la mise en contact d'une préparation de chondrocytes, notamment de chondrocytes humains, avec un milieu comprenant de l'albumine humaine, un polysaccharide et, éventuellement, une solution saline, le milieu étant dépourvu de DMSO et/ou de glycérol, et la congélation de la composition obtenue.

15. Méthode selon la revendication 13 ou 14, caractérisée en ce que la préparation de chondrocytes, notamment de chondrocytes humains, comprend au moins environ 5.10⁶ cellules/ml, avantageusement au moins environ 10⁷ cellules/ml.

16. Utilisation de chondrocytes obtenus par la méthode selon l'une des revendications 10 à 15 ou d'une composition selon l'une des revendications 5 à 9, pour la préparation d'une composition destinée à restaurer des défauts cartilagineux.

17. Méthode pour restaurer des défauts cartilagineux chez un sujet, comprenant l'implantation au sujet, dans la zone du défaut, d'une composition selon l'une des revendications 5 à 9.
